**Europäisches Patentamt**

**European Patent Office**

**Office européen des brevets**

(19)

(11) Veröffentlichungsnummer: **0 137 967**
**B1**

(12) **EUROPÄISCHE PATENTSCHRIFT**

(45) Veröffentlichungstag der Patentschrift:
02.07.86

(51) Int. Cl.⁴: **C 07 C 143/16, C 07 C 139/14**

(21) Anmeldenummer: 84109851.0

(22) Anmeldetag: 18.08.84

(54) Verfahren zur Aufarbeitung wässriger Lösungen von Natriummethallylsulfonat.

(30) Priorität: 12.10.83 DE 3337103

(43) Veröffentlichungstag der Anmeldung:
24.04.85 Patentblatt 85/17

(45) Bekanntmachung des Hinweises auf die Patenterteilung:
02.07.86 Patentblatt 86/27

(84) Benannte Vertragsstaaten:
BE CH DE FR GB IT LI NL

(56) Entgegenhaltungen:
CHEMICAL ABSTRACTS, Band 71, Nr. 12, 22.
September 1969, Seite 123, Nr. 51781d, Columbus,
Ohio, US;

(73) Patentinhaber: HÜLS AKTIENGESELLSCHAFT,
Patentabteilung / PB 15 - Postfach 13 20, D-4370
Marl 1 (DE)

(72) Erfinder: Müller, Dieter Jürgen, Dr., Stargarder
Strasse 30, D-4370 Marl (DE)
Erfinder: Austin, Severin, Dr., Am Gecksbach 30,
D-4270 Dorsten (DE)

## Description

Die Erfindung betrifft ein Verfahren zur Aufarbeitung wäßriger Lösungen von Natriummethallylsulfonat, wie sie bei der technischen Herstellung von Natriummethallylsulfonat (MAS) durch Umsetzung von Methallylchlorid (MAC) mit wäßriger $Na_2SO_3$-Lösung anfallen.

Bei diesem Verfahrensweg, der beispielsweise in der DE-PS 27 20 346 (= US-PS 4 171 324) beschrieben wird, entstehen wäßrige Lösungen, die Natriummethallylsulfonat und Natriumchlorid in nahezu äquimolaren Mengen enthalten, beispielsweise etwa 25 % MAS und etwa 10 % NaCl.

Zur Aufarbeitung dieser Lösungen und Abtrennung des MAS vom NaCl sind verschiedene Verfahren bekannt, bei denen es sich entweder um Extraktionsverfahren, beispielsweise gemäß US-PS 2 601 256 oder um Verfahren mit fraktionierter Kristallisation, beispielsweise gemäß DE-PS 22 21 736 (= US-PS 3 829 472) oder um Kombination dieser Verfahrensprinzipien, beispielsweise gemäß DE-PS 18 04 135 (= GB-PS 1 244 074) handelt.

Diesen Aufarbeitungsverfahren ist gemeinsam, daß die aufzuarbeitenden MAS-haltigen Lösungen zumindest in einem Teilschritt durch Eindampfen aufkonzentriert werden, wobei das Eindampfen sowohl unter vermindertem Druck, als auch bei Normal- oder Überdruck erfolgen kann. Dabei wird das in den einzudampfenden Lösungen enthaltene MAS je nach Eindampftemperatur und Eindampfdauer mehr oder weniger stark thermisch belastet, wobei es zu Nebenreaktionen mit Bildung von unerwünschten Nebenprodukten kommen kann.

Die thermische Belastung ist bei den Extraktionsverfahren im allgemeinen geringer, weil die notwendige Eindampfung bis zur Trockene vorzugsweise im Vakuum, also bei niedrigen Temperaturen, erfolgt.

Trotzdem haben die Extraktionsverfahren keine technische Bedeutung erlangt, weil diese den Einsatz großer Mengen an systemfremden Lösemitteln bzw. Extraktionsmitteln und eine apparativ und energetisch aufwendige Prozeßführung erfordern.

Demgegenüber hat die fraktionierte Kristallisation, wie sie beispielsweise in der DE-PS 22 21 736 beschrieben ist, trotz der größeren thermischen Belastung Eingang in die Technik gefunden, weil die Abtrennung des MAS von NaCl aus den wäßrigen Reaktionslösungen keine weiteren Hilfsstoffe erfordert und bezüglich der Prozeßführung einfacher zu handhaben ist.

Bei diesem Verfahren wird vorzugsweise unter Normaldruck bei Siedetemperaturen von etwa 100 bis 110 °C bis zu einem Gewichtsverhältnis der Salze zum verbliebenen Wasser von 1,3 bis 1,5 in der Lösung eingedampft, wobei zunächst nur der größte Teil des NaCl ohne Mitfällung von MAS auskristallisiert. Dies hängt damit zusammen, daß die Löslichkeit des NaCl in einer Lösung mit äquimolaren Mengen an MAS und NaCl unter 12 % liegt und nur wenig temperaturabhängig ist, während die Löslichkeit des MAS in solchen Lösungen mit steigender Temperatur stark zunimmt und bei 110 °C um etwa 50 % liegt. Kurz vor Beginn einer Mitfällung von MAS wird die Eindampfung unterbrochen, das ausgefallene NaCl heiß abgetrennt und das anfallende heiße Filtrat nach Zugabe von 0,1 bis 2 Gew.-% Wasser einer Kühlungskristallisation unterworfen, wobei nur MAS ohne Mitfällung des noch gelösten NaCl auskristallisiert. Bei dieser Kühlungskristallisation fällt um so mehr MAS aus, je höher die Endtemperatur bei der Eindampfung und je niedriger die Endtemperatur nach der Abkühlung ist. Deswegen ist man bestrebt, die Eindampfung vorzugsweise bei Siedetemperatur unter Normaldruck durchzuführen. Das auskristallisierte MAS wird in einem geeigneten Trennapparat wie beispielsweise einer Filterzentrifuge oder einer Nutsche als Feuchtkristallisat von der Mutterlauge abgetrennt. Auf diese Weise wird ein relativ NaCl-armes MAS-Kristallisat gewonnen, das durch zusätzliche Wasserwäsche noch weiter von NaCl befreit werden kann.

Dieses Verfahren arbeitet besonders wirtschaftlich wenn alle anfallenden Mutterlaugen und Waschwässer in die Eindampfstufe zurückgeführt werden, weil dann zumindest theoretisch das gesamte MAS als Kristallisat isolierbar ist. Durch diese Kreisfahrweise ist ein Teil des MAS einer sich ständig wiederholenden thermischen Belastung ausgesetzt. Infolgedessen reichern sich die unerwünschten Nebenprodukte in der Mutterlauge an, die das MAS-Kristallisat verunreinigen.

Bekanntlich wird zur Vermeidung von Verunreinigungen in Kristallisaten, die durch Nebenprodukt-haltige Mutterlaugen bedingt sind, in der Regel so verfahren, daß das Kristallisat ausreichend gewaschen wird und ein Teil der Waschwässer oder der Mutterlaugen ausgeschleust wird, d. h. nicht in den Kreislauf zurückgeführt, sondern verworfen wird. Auf diese Weise kann unter Verlust eines Teils des Zielproduktes, welches in diesem Fall MAS ist, der Gehalt an Nebenprodukten in den zurückzuführenden und im Kreislauf gefahrenen Mutterlaugen auf einem niedrigen, noch zulässigen Niveau weitgehend konstant gehalten werden.

Die Verluste an Zielprodukt durch Mutterlaugenausschleusung sind zwangsläufig um so größer, je größer die Bildungsgeschwindigkeit an Nebenprodukten bei der Eindampfung und Aufarbeitung der Reaktionslösung ist.

Damit stellt sich für die Reingewinnung von MAS aus MAS-/NaCl-haltigen Reaktionslösungen, Mutterlaugen und Waschwässern die grundsätzliche Aufgabe, ein Verfahren zur Eindampfung und Aufarbeitung der Lösungen zu finden, das die unerwünschte Nebenproduktbildung möglichst vollständig unterdrückt.

Die Aufgabe wird erfindungsgemäß entsprechend den Angaben der Patentansprüche gelöst.

Überraschenderweise wurde nun gefunden, daß man die Nebenproduktbildung bei der thermischen Belastung durch Eindampfung von wäßrigen Lösungen von Natriummethallylsulfonat mit etwa äquimolarem NaCl-Gehalt (etwa 25 % MAS und etwa 10 % NaCl) erheblich vermindern kann, wenn während der Eindampfung der pH-Wert der Lösung, bzw. der sich bildenden Suspension im Bereich von 7,5 bis 10,5, vorzugsweise von 8,5 bis 9,5, gehalten wird, wobei sich die pH-Werte auf die jeweilige Eindampftemperatur beziehen.

Die Eindampfung erfolgt in einem Temperaturbereich von 75 bis 110 °C, bevorzugt von 85 bis 95 °C, vorzugsweise unter vermindertem Druck.

Da MAS und NaCl als Salze aus starken Säuren und starken Basen in wäßriger Lösung neutral reagieren und sich bei Raumtemperatur ein pH-Wert von etwa 7 einstellt, der mit steigender Temperatur noch abfällt, war nicht zu erwarten, daß es vorteilhafter ist, solche MAS-/NaCl-haltigen Lösungen schwach alkalisch zu stellen und im alkalischen Milieu in einem bestimmten pH-Bereich einzudampfen.

Bei einer Eindampfung unterhalb pH 7 neigt MAS überraschenderweise verstärkt zur Nebenproduktbildung, wobei vermutlich bevorzugt Dimere bzw. Oligomere des MAS entstehen. Diese Vermutung wird gestützt durch KMR-Analysen an MAS-haltigen Lösungen, die im neutralen bis schwachsauren Milieu thermisch belastet worden sind. Es hat sich bei der Auswertung derartiger KMR-Spektren gezeigt, daß die Tendenz zur Dimerenbildung bei Erhöhung des pH-Wertes um eine Einheit unter sonst gleichen Bedingungen um den Faktor von etwa 2,5 abnimmt. Demnach wäre es eigentlich anzustreben, die Eindampfung von MAS-haltigen Lösungen bei möglichst hohen pH-Werten vorzunehmen.

Es wurde jedoch weiter gefunden, daß der Anhebung des pH-Wertes dadurch Grenzen gesetzt sind, daß mit zunehmendem pH-Wert die Bildung eines anderen unerwünschten Nebenproduktes zum Tragen kommt. Bei diesem Nebenprodukt handelt es sich aufgrund von KMR-Analysen mit großer Wahrscheinlichkeit um Isocrotylsulfonat (ICS), welches sich durch Doppelbindungsisomerisierung aus MAS bei pH-Werten oberhalb etwa 9,5 zu bilden beginnt.

Eine Erhöhung des pH-Wertes um eine Einheit erhöht die Bildungsgeschwindigkeit des ICS unter sonst gleichen Bedingungen um den Faktor von etwa 10.

Um sowohl die vermutete Dimerisierung als auch die Isomerisierung des MAS gering zu halten, ist daher ein pH-Bereich von 7,5 bis 10,5, vorzugsweise von 8,5 bis 9,5 bei der Eindampfung einzustellen und konstant zu halten. Überraschenderweise hat sich gezeigt, daß der eingestellte pH-Wert von MAS-haltigen Lösungen bei der thermischen Belastung im allgemeinen nicht konstant bleibt, sondern asymptotisch um 1 bis 1,5 pH-Einheiten abfällt. Es ist nicht bekannt, worauf dieser pH-Abfall beruht, denn sowohl bei einer Dimerisierung wie bei einer Isomerisierung werden keine Hydroxylionen verbraucht. Im allgemeinen ist es daher zur Aufrechterhaltung der erfindungsgemäßen pH-Wert-Bereiche erforderlich, während der gesamten Eindampfzeit geringe Mengen einer basischen Lösung, beispielsweise einer NaOH-Lösung, zuzudosieren.

Es ist zudem gefunden worden, daß die Grenzen der einzuhaltenden pH-Bereiche vorzugsweise um so enger zusammen liegen sollen, je höher die Eindampftemperatur ist. Das heißt bei höheren Eindampftemperaturen, bei denen die Bildungsgeschwindigkeit der Nebenprodukte naturgemäß größer ist, ist der einzuhaltende pH-Bereich besonders eng. Beispielsweise sollte bei Eindampfung unter Normaldruck und einer Eindampftemperatur von etwa 100 °C bis etwa 110 °C möglichst ein pH-Wert um 9,0 ± 0,5 eingehalten werden, während beispielsweise bei Eindampfung unter vermindertem Druck bei einer Eindampftemperatur von 85 bis 100 °C, vorzugsweise etwa 90 °C, die Einhaltung eines pH-Wertes von 9,0 ± 1,0 ausreichend ist. Bei einer Eindampftemperatur von 75 bis 85 °C, vorzugsweise etwa 80 °C, genügt die Einhaltung eines pH-Wertes von etwa 9,0 - 1,5. Die vorstehend genannten pH-Bereiche können zwar nach beiden Seiten ausgedehnt werden, doch muß dann mit vermehrter Nebenproduktbildung gerechnet werden, was gegebenenfalls in Kauf genommen werden kann, wenn die Eindampfzeiten kurz sind.

Wegen des bei niedrigeren Eindampftemperaturen größeren Spielraums bei der pH-Einstellung und -Regelung und der verminderten Bildungsgeschwindigkeit der Nebenprodukte ist es vorteilhaft, die Eindampfung vorzugsweise unter vermindertem Druck bei Temperaturen bis 100 °C vorzunehmen.

Allerdings kann wegen der mit abnehmender Temperatur sinkenden Löslichkeit von MAS die Eindampftemperatur nicht beliebig abgesenkt werden, weil sonst bei der Kühlungskristallisation nach dem Eindampfen entsprechend weniger MAS auskristallisiert und damit mehr MAS mit den Mutterlaugen im Kreis gefahren werden muß. Man stellt daher eine Eindampftemperatur von 75 °C bis 110 °C, vorzugsweise von etwa 85 °C bis 95 °C, ein und dampft die Lösungen bis zu einem Gewichtsverhältnis der gelösten Salze zum verbliebenen Wasser von 0,8 bis 1,5 in der Lösung ein.

Anschließend trennt man das auskristallisierte NaCl heiß, vorzugsweise bei der Eindampftemperatur, ab und kühlt das Filtrat zur Kristallisation des MAS, vorzugsweise auf Temperaturen von 10 bis 30 °C, insbesondere 15 bis 25 °C. Nach dem Abtrennen des MAS führt man das Filtrat in den Eindampfprozeß zurück.

Im allgemeinen führt man nur den größten Teil des Filtrats in den nachfolgenden Eindampfprozeß zurück. Einen kleinen Teil, beispielsweise 2 bis 20 % der Mutterlauge, schleust man gegebenenfalls aus. Die ausgeschleuste Menge richtet sich nach dem Gehalt an Dimerem und Isomerem in der Mutterlauge.

Vorteilhaft ist eine Eindampfung in zwei oder mehr Druckstufen, wobei zunächst unter Einhaltung des erfindungsgemäßen pH-Bereiches die Hauptmenge an Wasser unter vermindertem Druck, vorzugsweise 250 bis 750 mbar, bei Temperaturen von 75 bis 100 °C, vorzugsweise 85 bis 95 °C, soweit abgedampft wird, daß neben dem auskristallisierenden NaCl eben noch kein MAS ausfällt. Dies wird je nach Eindampftemperatur bei einem Gewichtsverhältnis der gelösten Salze zum verbleibenden Wasser von 0,8 bis 1,3 erreicht. Danach wird bei höherem Druck, vorzugsweise bei Normaldruck im Temperaturbereich von 100 °C bis 110 °C die restliche Menge an Wasser ebenfalls unter Einhaltung des erfindungsgemäßen pH-Bereiches bis zur gewünschten Endkonzentration, jedoch höchstens soweit abgedampft, daß die Löslichkeitsgrenze des MAS nicht überschritten wird. Dies ist bei einem Gewichtsverhältnis der gelösten Salze zum verbleibenden Wasser von 1,3 bis 1,5 der Fall.

Diese Prozeßführung hat den Vorteil, daß die Haupteindampfung bei niederer Temperatur im erfindungsgemäßen pH-Bereich ohne nennenswerte Nebenproduktbildung durchgeführt werden kann und nur die Resteindampfung bei höherer Temperatur ebenfalls im erfindungsgemäßen pH-Bereich durchgeführt wird, um einerseits die Nebenproduktbildung auch bei höheren Temperaturen so gering wie möglich zu halten und andererseits wegen der größeren Löslichkeit des MAS bei höheren Temperaturen die Lösung möglichst hoch aufkonzentrieren zu können.

Auf diese Weise wird gleichzeitig eine Minimierung der Nebenproduktbildung und eine Maximierung des bei der nachfolgenden Kühlungskristallisation anfallenden MAS-Kristallisates erreicht. Zudem erhält man sehr reines Natriummethallylsulfonat (über 99,8 %ig).

Die folgenden Beispiele sollen das Verfahren näher erläutern.

**Beispiel 1**

In einem 3 m³ fassenden Rührbehälter mit Mantelheizung im unteren Drittel des Behälters und Umpumpvorrichtung werden 2 500 kg einer Lösung mit 27,4 % MAS und 10,4 % NaCl vorgelegt, unter Rühren bei Normaldruck auf 90 °C erwärmt und bei dieser Temperatur mittels Zugabe von Natronlauge auf pH 9 eingestellt. Danach wird der Druck soweit auf etwa 500 mbar erniedrigt, daß die Lösung zu sieden beginnt. Bei dieser Temperatur wird unter Einhaltung des pH-Wert-Bereiches von 8,5 bis 9,5 durch weitere Zugabe geringer Mengen an Natronlauge eingedampft.

Die abgeführten Wassermengen werden messend verfolgt. Nach Abdampfen von etwa 440 kg Wasser beginnt die Kristallisation von NaCl. Die Eindampfung der sich bildenden Suspension wird bei 90 °C im pH-Bereich von 8,5 bis 9,5 fortgesetzt, bis etwa 820 kg Wasser abgedampft sind. Das abgedampfte Wasser entspricht einem Mengenanteil von ca. 33 % der ursprünglichen Lösungsmenge. An diesem Punkt wird die Eindampfung bei 90 °C abgebrochen, um eine Mitfällung von MAS zu vermeiden. Im Normalfall kann an diesem Punkt das auskristallisierte NaCl heiß bei mindestens 90 °C abgenutscht und das Filtrat zur Kristallisation des MAS von 90 °C auf etwa 20 °C abgekühlt werden. Dabei kristallisieren etwa 420 kg MAS aus.

In der bevorzugten Ausführungsform (nach Anspruch 4) wird der Druck jedoch auf Normaldruck erhöht und bei einem Siedepunkt von etwa 110 °C weitere 120 kg Wasser abgedampft, entsprechend einer gesamten Wassermenge von etwa 38 % der ursprünglichen Lösungsmenge. Dabei kristallisieren etwa 160 kg NaCl ohne Mitfällung von MAS aus. Das auskristallisierte NaCl wird in der Siedehitze abgenutscht, das Filtrat in einen Kristallisator übergeführt und das NaCl-Kristallisat mit Wasser weitestgehend MAS-frei gewaschen. Die Waschwässer werden in den nachfolgenden Eindampfprozeß zurückgeführt. Das in den Kristallisator übergeführte Filtrat wird einer Kühlungskristallisation auf etwa 20 °C unterworfen. Dabei kristallisieren etwa 460 kg MAS aus, während NaCl mit dem restlichen MAS gelöst bleibt. Das Filtrat wird zum größten Teil in den nachfolgenden Eindampfprozeß zurückgeführt. Ein kleinerer Teil wird ausgeschleust, um eine Anreicherung von Nebenprodukten in den im Kreislauf geführten Mutterlaugen zu vermeiden. Die auszuschleusende Teilmenge richtet sich nach dem Gehalt an Dimeren bzw. Isomeren in der im Kreislauf geführten Mutterlauge. Unter den in diesem Beispiel gewählten Parametern genügt im Dauerbetrieb eine Ausschleusung von etwa 5 % der Mutterlauge, was einem MAS-Verlust von etwa 2 % der Gesamtmenge entspricht. Somit sind unter den erfindungsgemäßen Bedingungen im Dauerbetrieb etwa 98 % des MAS in reiner Form isolierbar.

Das MAS-Kristallisat wird mittels Filterzentrifuge kalt filtriert und mit kaltem Wasser weitestgehend NaCl-frei gewaschen. Auch diese Waschwässer werden in den nachfolgenden Eindampfprozeß zurückgeführt. Man erhält reines 99 %iges MAS.

## Beispiel 2

In einem 3 m³ fassenden Rührbehälter mit Mantelheizung im unteren Drittel des Behälters und Umpumpvorrichtung legt man jeweils 2 500 kg einer Lösung mit 25,8 % MAS und 10,1 % NaCl vor, erwärmt bei Normaldruck auf die in Tabelle 1 angegebenen Temperaturen (75, 90 und 105 °C) und stellt durch Zugabe von Natronlauge die in Tabelle 1 angegebenen pH-Werte (8,0; 9,0; 10,0 und 10,5) ein. Nun wird der Druck jeweils soweit vermindert, daß die Lösungen bei den jeweiligen Temperaturen sieden und durch verdampfen aufkonzentriert werden können.

Während des Eindampfens hält man die angegebenen pH-Werte durch weitere Zugabe geringer Mengen an Natronlauge weitgehend konstant ( ± 0,5). Das Eindampfen wird solange fortgesetzt, bis die Löslichkeitsgrenze des Methallylsulfonates für die angegebenen Temperaturen erreicht ist. Man bricht die Eindampfung ab und trennt das auskristallisierte NaCl heiß bei der Eindampftemperatur ab. Das Filtrat kühlt man zur Kristallisation des MAS auf 20 °C und trennt das auskristallisierte MAS ab.

Die anfallende Mutterlauge führt man in den Eindampfprozeß zurück, wobei man gegebenenfalls (s. Tabelle 1) geringe Mengen ausschleust. Das MAS-Kristallisat wird mit kaltem Wasser derart gewaschen, daß dieses einen restlichen Chloridgehalt von weniger als 0,1 % aufweist. Das Waschwasser wird in die Eindampfstufe zurückgeführt.

Nach 5maligem Kreislauf zieht man vor dem Abtrennen des NaCl Proben aus der Lösung und untersucht durch KMR-Analyse auf Nebenprodukte.

Dabei wird das KMR-Signal bei 1,3 ppm dem Dimerisat, das KMR-Signal bei 6,1 ppm dem Isocrotylsulfonat und das KMR-Signal bei 1,9 ppm dem MAS zugeordnet. Die chemischen Verschiebungen beziehen sich auf einen inneren Standard. Die Auswertung erfolgt in relativen Flächeneinheiten, die den tatsächlichen molaren Konzentrationen an Nebenprodukten bezogen auf das MAS weitgehend entsprechen.

Die bei unterschiedlichen Eindampftemperaturen und pH-Werten erhaltenen Ausbeuten an MAS im Dauerbetrieb bei Ausschleusung der angegebenen Mengen an Mutterlauge je Durchgang sind in Tabelle 1 angegeben.

## Tabelle 1

Ausbeuten (%) an MAS im Dauerbetrieb, Nebenproduktbildung an Dimeren (DIM) [Mol-%] bzw. Isomeren (ISO) [mol-%] bei verschiedenen Eindampftemperaturen und pH-Werten nach 5maligem Kreislauf der Mutterlaugen und Waschwässer sowie ausgeschleuster Anteil an Mutterlauge (Ausg.A.) [%] je Durchgang.

| pH | 8,0 | | | | 9,0 | | | | 10,0 | | | | 10,5 | | | |
|---|---|---|---|---|---|---|---|---|---|---|---|---|---|---|---|---|
| Temp. °C | Dim Mol-% | Iso Mol-% | Ausg.A %  | Ausb. % | Dim Mol-% | Iso Mol-% | Ausg.A % | Ausb. % | Dim Mol-% | Iso Mol-% | Ausg.A % | Ausb. % | Dim Mol-% | Iso Mol-% | Ausg.A % | Ausb. % |
| 75 | — | — | 2 | 99 | — | — | 0 | >99 | — | — | 2 | 99 | — | 1,5 | 5 | 98 |
| 90 | — | — | 2 | 99 | — | — | 2 | 99 | — | 1,5 | 5 | 98 | — | 4,0 | 8 | 97 |
| 105 | 1,5 | — | 5 | 98 | 0,5 | 1,0 | 5 | 98 | — | 5 | 10 | 96 | — | 10 | 20 | 92 |

## Vergleichsbeispeil A

Bei der Durchführung eines Versuches mit gleichem Ansatz wie im Beispiel 1, wobei jedoch von Beginn an unter Normaldruck im Temperaturbereich von 100 bis 110 °C eingedampft worden ist und der pH-Wert der Lösung im Normalbereich von etwa 5 bis 7 gelegen hat, hat sich bei der Eindampfung der Lösung bzw. der sich bildenden Suspension bis zu etwa gleichem Endzustand wie im Beispiel 1 gezeigt, daß im Dauerbetrieb etwa 25 % der Mutterlaugen ausgeschleust werden müssen.

Das entspricht im Dauerbetrieb einem MAS-Verlust von etwa 10 % der ursprünglich vorhandenen Gesamtmenge, so daß in diesem Fall nur 90 % des MAS in reiner Form isolierbar sind.

## Patentansprüche

1. Verfahren zur Aufarbeitung wäßriger Lösungen von Natriummethallylsulfonat mit etwa äquimolarem NaCl-Gehalt, wie sie bei der Umsetzung von Methallylchlorid mit wäßriger $Na_2SO_3$-Lösung anfallen,

dadurch gekennzeichnet,

daß man die Lösungen nach Einstellung und unter Einhaltung eines pH-Bereiches von 7,5 bis 10,5 im Temperaturbereich von 75 bis 110 °C bis knapp unter die Löslichkeitsgrenze des Methallylsulfonates eindampft, wobei nur NaCl auskristallisiert, und nach Abtrennen des NaCl-Kristallisates das heiße Filtrat abkühlt, wobei nur Natriummethallylsulfonat als Kristallisat anfällt, welches abgetrennt wird, und die dabei anfallende Mutterlauge in den Eindampfprozeß zurückführt, wobei man gegebenenfalls einen kleinen Teil der Mutterlauge, bis 20 %, ausschleust.

2. Verfahren nach Anspruch 1, dadurch gekennzeichnet, daß man einen pH-Bereich von 8,5 bis 9,5 einhält.

3. Verfahren nach Anspruch 1 und 2, dadurch gekennzeichnet, daß man in einem Temperaturbereich von 85 bis 95 °C eindampft.

4. Verfahren nach Anspruch 1, dadurch gekennzeichnet, daß man bei Eindampftemperaturen von 100 bis 110 °C und Normaldruck einen pH-Bereich von 9,0 ± 0,5, bei einer Eindampftemperatur von 85 bis 100 °C und vermindertem Druck einen pH-Bereich von 9,0 ± 1,0 und bei einer Eindampftemperatur von 75 bis 85 °C und vermindertem Druck einen pH-Bereich von 9,0 ± 1,5 einhält.

5. Verfahren nach den Ansprüchen 1 bis 4, dadurch gekennzeichnet, daß man in zwei oder mehr Druckstufen eindampft, wobei zunächst unter vermindertem Druck bei 75 bis 100 °C, vorzugsweise 85 bis 95 °C, die überwiegende Menge des abzudampfenden Wassers entfernt wird und danach unter Normaldruck bei Temperaturen von 100 bis 110 °C die Restmenge an abzudampfenden Wasser entfernt wird.

## Claims

1. A process for working up an aqueous solution of sodium methallylsulphonate containing an approximately equimolar amount of NaCl, such as arises in the conversion of methallyl chloride with an aqueous Na$_2$SO$_3$ solution, characterised in that the solution is evaporated down at a temperature in the range of from 75 to 110°C to near to the solubility limit of the methallylsulphonate after reaching and during maintenance of a pH in the range of 7.5 to 10.5, so that only NaCl crystallizes out, the hot filtrate is cooled after separation of the NaCl crystals, so that only sodium methallylsulphonate precipitates as crystals which are separated out, and the resulting mother liquor is returned to the evaporation process, optionally with the exception of a small proportion, up to 20%, of the mother liquor.

2. A process according to claim 1, characterisedf in that a pH in the range of 8.5 to 9.5 is maintained.

3. A process according to claim 1 or 2, characterised in that the evaporation is carried out at a temperature in the range 85 to 95°C.

4. A process according to claim 1, characterised in that the pH is maintained either in the range of 9.0 ± 0.5 at an evaporation temperature of 100 to 110°C and normal pressure, or in the range of 9.0 ± 1.0 at an evaporation temperature of 85 to 100°C and reduced pressure, or in the range of 9.0 + 1.5 at an evaporation temperature of 75 to 85°C and reduced pressure.

5. A process according to any of claims 1 to 4, characterised in that the evaporation is carried out in two or more pressure stages, the major proportion of the water to be evaporated being removed first under reduced pressure at 75 to 100°C, preferably 85 to 95°C and then the remainder of the water to be evaporated being removed under normal pressure at a temperature of 100 to 110°C.

## Revendications

1. Procédé pour le traitement de solutions aqueuses de méthallyl-sulfonate de sodium ayant une teneur à peu près équimolaire en NaCl, telles qu'elles se forment lors de la réaction du chlorure de méthallyle sur une solution aqueuse de Na$_2$SO$_3$, caractérisé par le fait qu'après avoir établi et en maintenant une gamme de pH de 7,5 à 10,5, dans l'intervalle de température de 75 à 110°C, on évapore les solutions jusqu'à peine endessous de la limite de solubilité du méthallyl-sulfonate, NaCl seul se séparant par cristallisation, et qu'après avoir séparé le produit de cristallisation NaCl, on refroidit le filtrat chaud, le méthallyl-sulfonate de sodium se formant seul comme produit de cristallisation que l'on sépare, et que l'on ramène au processus d'évaporation la liqueur mere, en éliminant éventuellement par éclusage une petite partie de la liqueur mère, jusqu'à 20 %.

2. Procédé selon la revendication 1, caractérisé par le fait que l'on maintient une gamme de pH de 8,5 à 9,5.

3. Procédé selon la revendication 1 et 2, caractérisé par le fait que l'on évapore dans un intervalle de température de 85 à 95°C.

4. Procédé selon la revendication 1, caractérisé par le fait qu'à des températures d'évaporation de 100 à 110°C et à la pression normale on maintient une gamme de pH de 9,0 ± 0,5, à une température d'évaporation de 85 à 100°C et sous pression réduite une gamme de pH de 9,0 ± 1,0 et à une température d'évaporation de 75 à 85°C et sous pression réduite, une gamme de pH de 9,0 ± 1,5.

5. Procédé selon les revendications 1 à 4, caractérisé par le fait que l'on évapore en deux ou plusieurs étapes de pression, en éliminant tout d'abord sous pression réduite, à une température de 75 à 100°C, de préférence de 85 à 95°C, la majeure partie de l'eau à évaporer et qu'ensuite sous une pression normale à des températures de 100 à 110°C, on élimine la quantité résiduelle d'eau à évaporer.